(19) Europäisches Patentamt
European Patent Office
Office européen des brevets

(11) Veröffentlichungsnummer: **0 409 026 A1**

(12) **EUROPÄISCHE PATENTANMELDUNG**

(21) Anmeldenummer: **90113022.9**

(51) Int. Cl.[5]: **C07D 231/06**, A01N 43/56

(22) Anmeldetag: **07.07.90**

(30) Priorität: **20.07.89 DE 3924112**

(43) Veröffentlichungstag der Anmeldung:
**23.01.91 Patentblatt 91/04**

(84) Benannte Vertragsstaaten:
**AT BE DE DK ES FR GB IT NL**

(71) Anmelder: **BAYER AG**

**D-5090 Leverkusen 1 Bayerwerk(DE)**

(72) Erfinder: **Kleefeld, Gerd, Dr.**
**Otto-Hahn-Strasse 87**
**D-4000 Düsseldorf 13(DE)**
Erfinder: **Dutzmann, Stefan, Dr.**
**Leinenweberweg 33**
**D-4000 Düsseldorf 13(DE)**

(54) **Fungizide Mittel auf Basis von 1,3,5-Triaryl-2-pyrazolin-Derivaten, neue 1,3,5-Triaryl-2-pyrazoline und ein Verfahren zu ihrer Herstellung.**

(57) Verwendung von teilweise bekannten 1,3,5-Triaryl-2-pyrazolin-Derivaten der Formel (I)

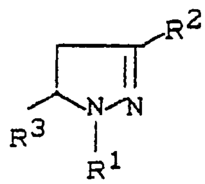

(I)

in welcher
$R^1$-$R^3$ die in der Beschreibung angegebene Bedeutung haben, zur Bekämpfung von Pilzen und einige noch neue Verbindungen.

Die neuen und bekannten Verbindungen können nach Analogieverfahren hergestellt werden, z.B. indem man geeignete Enone mit geeigneten Hydrazinen umsetzt.

EP 0 409 026 A1

## FUNGIZIDE MITTEL AUF BASIS VON 1,3,5-TRIARYL-2-PYRAZOLIN-DERIVATEN, NEUE 1,3,5-TRIARYL-2-PYRAZOLINE UND EIN VERFAHREN ZU IHRER HERSTELLUNG

Die Erfindung betrifft die Verwendung von teilweise bekannten 1,3,5-Triaryl-2-pyrazolin-Derivaten zur Bekämpfung von Schädlingen, neue 1,3,5-Triaryl-2-pyrazolin-Derivate und ein Verfahren zu ihrer Herstellung.

Es ist bereits bekannt, daß bestimmte 1,3,5-Triaryl-2-pyrazolin-Derivate als Spasmolytika oder als Ausgangsstoffe bei der Herstellung pharmazeutischer Wirkstoffe Verwendung finden (vergleiche Res. Commun. Chem. Pathol Pharmacol. 56 (1), 129 - 32 (1987) Zitat in CA 107 , 51823 f; J. Pharm Sci. 76 (8), 626 - 32 (1987) Zitat in CA 107 , 228455 r).

Weiterhin sind die Synthesen von zahlreichen 1,3,5-Triaryl-2-pyrazolin-Derivaten beschrieben (vgl. u. a. Can. J. Chem. 57 (3), 360 - 66 (1979); Aust. 3. Chem. 32 (7), 1601 - 12 (1979); Pharmazie 36 (11), 754 - 56 (1981); Khim, Geterosikl. Soedin. 7 , 965 - 68 (1984); J. Chin Chem. Soc. (Taipei 31 (4), 383 - 90 (1984); Rev. Roum. Chim. 31 (6), 629 - 35 (1986)), über deren Wirksamkeit im Pflanzenschutz ist jedoch nichts bekannt.

Ferner sind zahlreiche 1,3,5-Triaryl-2-pyrazolin-Derivate als Farbstoffe und optische Aufheller bekannt (vergleiche unter anderem Ukr. Khim. Zh. 45 (6), 553 - 56 (1979); Aust. J. Chem. 30 (3), 629 - 37 (1977); Nippon Kagaku Kaishi 2 , 271 - 75 (1978); CH-609977; DE 27 49 982; DE 26 44 286; DE 25 50 548).

Es ist weiter bereits bekannt, daß 4-(2-Pyrazolin-3-yl)-phenylaminophenyl-sulfide und -sulfone bakterizide Eigenschaften besitzen (vergleiche Egypt. J. Pharm. Sci. 27 (1-4), 43 - 58 (1986) Zitat in CA 107 , 175983c).

Außerdem weiß man, daß bestimmte 1,3,5-Triaryl-2-pyrazolin-Derivate, wie beispielsweise das 1-(4-Chlorphenyl)-3-(3,4-dichlorphenyl)-5-phenyl-2-pyrazolin, die Aflatoxin Produktion von Aspergillus flavus hemmen (vergleiche Pestic. Sci. 16 , 147 - 51 (1985)).

Von einigen 1,3,5-Triaryl-2-pyrazolin-Derivaten, wie beispielweise dem 1,5-Diphenyl-3-(4-carboxyphenyl)-2-pyrazolin, ist eine bakterizide und fungizide in vitro Wirksamkeit beschrieben (vergleiche Egypt. J. Chem. 25 (3), 293 - 300 (1982)). Über deren Anwendung im Pflanzenschutz ist nichts bekannt.

Auch die insektizide Wirksamkeit einiger 1,3,5-Triaryl2-pyrazolin-Derivate, wie beispielsweise des 3,4-(4-Fluorphenyl)-1-(4-trifluormethoxyphenyl)carbamoyl-pyrazolin, ist dokumentiert (vergleiche EP 0 267 869).

Es wurde gefunden, daß die teilweise bekannten 1,3,5-Triaryl-2-pyrazolin-Derivate der allgemeinen Formel (I),

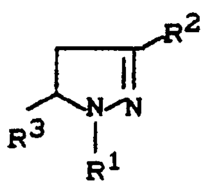

(I)

in welcher

$R^1$, $R^2$ und $R^3$ unabhängig voneinander jeweils für gegebenenfalls einfach oder mehrfach, gleich oder verschieden substituiertes Phenyl stehen, wobei als Substituenten in Frage kommen:

Halogen, Hydroxy, Mercapto, Nitro, Amino, Sulfo ($-SO_3H$), geradkettiges oder verzweigtes Alkyl mit 1 bis 4 Kohlenstoffatomen, geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen, geradkettiges oder verzweigtes Alkoxy oder Alkylthio mit jeweils 1 bis 4 Kohlenstoffatomen, (Di)alkylamino mit 1 bis 4 Kohlenstoffatomen in den jeweiligen geradkettigen oder verzweigten Alkylteilen, Carboxyl, geradkettiges oder verzweigtes Alkoxycarbonyl mit 1 bis 8 Kohlenstoffatomen im Alkoxyteil, geradkettiges oder verzweigtes Acyloxy mit 1 bis 8 Kohlenstoffatomen.

Weiterhin kommen als Substituenten jeweils gegebenenfalls einfach oder mehrfach, gleich oder verschieden substituiertes Phenyl, Phenoxy oder Phenoxycarbonyl in Frage, wobei als Substituenten die oben aufgeführten Substituenten für Phenyl genannt seien,

fungizide Eigenschaften besitzen.

Die erfindungsgemäß zu verwendenden 1,3,5-Triaryl-2-pyrazolin-Derivate der Formel (I) besitzen ein asymmetrisch substituiertes Kohlenstoffatom. Die Erfindung betrifft sowohl die einzelnen Isomeren als auch deren Gemische.

Überraschenderweise zeigen die erfindungsgemäß zu verwendenden 1,3,5-Triaryl-2-pyrazolin-Derivate der Formel (I) eine bessere fungizide Wirksamkeit als das aus dem Stand der Technik bekannte 3,4-(4-Fluorphenyl)-1-(4-trifluormethoxyphenyl)-carbamoyl-pyrazolin, welches eine chemisch naheliegende Verbindung ist.

Die erfindungsgemäß zu verwendenden 1,3,5-Triaryl-2-pyrazolin-Derivate sind durch die Formel (I) allgemein definierte. Bevorzugt sind diejenigen Verbindungen der Formel (I), bei welchen

$R^1$, $R^2$ und $R^3$ unabhängig voneinander jeweils für gegebenenfalls einfach bis dreifach, gleich oder verschieden substituiertes Phenyl stehen, wobei als Substituenten in Frage kommen:

Fluor, Chlor, Brom, Hydroxy, Mercapto, Nitro, Amino, Sulfo ($-SO_3H$), geradkettiges oder verzweigtes Alkyl mit 1 bis 3 Kohlenstoffatomen; Halogenalkyl, Halogenalkoxy oder Halogenalkylthio mit jeweils 1 oder 2 Kohlenstoffatomen und jeweils 1 bis 5 Fluor- und/oder Chloratomen, geradkettiges oder verzweigtes Alkoxy oder Alkylthio mit jeweils 1 bis 3 Kohlenstoffatomen, Alkylamino oder Dialkylamino mit 1 oder 2 Kohlenstoffatomen in den jeweiligen Alkylteilen, Carboxyl, geradkettiges oder verzweigtes Alkoxycarbonyl mit 1 bis 6 Kohlenstoffatomen im Alkoxyteil, geradkettiges oder verzweigtes Acyloxy mit 1 bis 6 Kohlenstoffatomen.

Weiterhin kommen als Substituenten jeweils gegebenenfalls einfach bis dreifach, gleich oder verschieden substituiertes Phenyl, Phenoxy oder Phenoxycarbonyl in Frage, wobei als Substituenten die als bevorzugt für Phenyl aufgeführten Substituenten genannt seien.

Besonders bevorzugt sind diejenigen Verbindungen der Formel (I), bei welchen

$R^1$, $R^2$ und $R^3$ unabhängig voneinander jeweils für gegebenenfalls einfach oder zweifach, gleich oder verschieden substituiertes Phenyl stehen, wobei als Substituenten in Frage kommen:

Fluor, Chlor, Brom, Hydroxy, Mercapto, Nitro, Amino, Sulfo ($-SO_3H$), Methyl, Ethyl, Trifluormethyl, Trifluormethoxy, Trifluormethylthio, Methoxy, Methylthio, Methylamino, Dimethylamino, Carboxyl, Methoxycarbonyl, Ethoxycarbonyl, n- oder i-Propoxycarbonyl, n-, s-, i- oder t-Butoxycarbonyl, Formyloxy, Acetyloxy, Propionyloxy, n-Butyryloxy oder i-Butyryloxy.

Weiterhin kommen als Substituenten jeweils gegebenenfalls einfach oder zweifach, gleich oder verschieden substituiertes Phenyl, Phenoxy oder Phenoxycarbonyl in Frage, wobei als Substituenten die als besonders bevorzugt aufgeführten Substituenten für Phenyl genannt seien.

Die erfindungsgemäß zu verwendenden 1,3,5-Triaryl-2-pyrazolin-Derivate der Formel (I) sind teilweise bekannt.

Noch nicht bekannt sind folgende 1,3,5-Triaryl-2-pyrazolin-Derivate.

1- (4-Chlorphenyl )-3-phenyl-5-(4-hydroxyphenyl )-2-pyrazolin, 1-(4-Methylphenyl)-3-phenyl-5-(4-hydroxyphenyl)-2-pyrazolin und 1-(4-Methoxyphenyl)-3-phenyl-5-(4-hydroxyphenyl)-2-pyrazolin, die ebenfalls Teil der Erfindung sind und durch die allgemeinen Formel (IA)

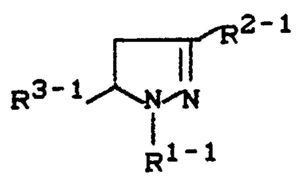

(IA)

in welcher

$R^{1-1}$ für 4-Chlorphenyl, 4-Methylphenyl oder 4-Methoxyphenyl steht,

$R^{2-1}$ für Phenyl steht und

$R^{3-1}$ für 4-Hydroxyphenyl steht, beschrieben werden.

Die oben genannten neuen und auch bekannten Verbindungen können in Analogie zu bekannten Verfahren nach üblichen Methoden synthetisiert werden (vergleiche unter anderem Can. J. Chem. 57 (3), 360 - 66 (1979); Indian J. Chem. 19 B (5), 364 - 67 (1980)). Man erhält diese neuen 1,3,5-Triaryl-2-pyrazolin-Derivate der Formel (IA) z.B., indem man Enone der Formel (II)

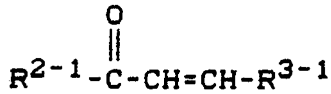

(II)

in welcher

$R^{2-1}$ für Phenyl steht und
$R^{3-1}$ für 4-Hydroxyphenyl steht,
mit Hydrazinen der Formel (III)
$H_2N\text{-}NH\text{-}R^{1-1}$     (III)
in welcher
$R^{1-1}$ für 4-Chlorphenyl, 4-Methylphenyl oder 4-Methoxyphenyl steht,
gegebenenfalls in Gegenwart eines Verdünnungsmittels wie beispielsweise Essigsäure und gegebenenfalls in Gegenwart eines Katalysators wie beispielsweise Schwefelsäure bei Temperaturen zwischen 20 °C und 150 °C kondensiert.

Verwendet man beispielsweise 1-(4-Chlorphenyl)-3-(4-hydroxyphenyl)-2-propen-1-on und Phenylhydrazin als Ausgangsstoffe, sc läßt sich der Reaktionsablauf zur Herstellung der neuen Verbindungen durch das folgende Formelschema darstellen:

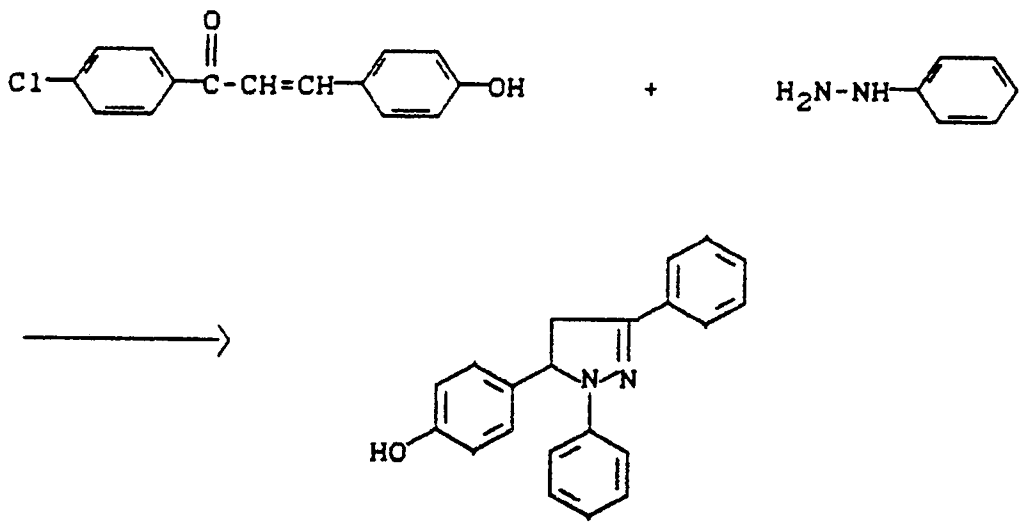

Enone der Formel (II) und Hydrazine der Formel (III) sind allgemein bekannte Verbindungen der organischen Chemie.

Die erfindungsgemäß zu verwendenden Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;

Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei eignen sich die erfindungsgemäß zu verwendenden Wirkstoffe insbesondere zur Bekämpfung von Gerstenmehltau (Erysiphe graminis f. sp. hordei) und zur protektiven Anwendung gegen Mehltau an Weizen (Erysiphe graminis f. sp. tritici).
Außerdem zeigen einige der erfindungsgemäß zu verwendenden Wirkstoffe auch fungizide Wirkung gegen Leptosphaeria nodorum und Pyrenophora teres an Getreide, gegen Mehltau an Gurken (Sphaerotheca) und Reben (Uncinula) sowie gegen Pyricularia an Reis.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenak tiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabak stengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B.Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Ole sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß zu verwendenden Wirkstoffe können in den Formulierungen in Mischung mit anderen bekannten Wirkstoffen vorliegen wie Fungizide, Insektizide, Akari zide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die Herstellung und Anwendung der erfindungsgemäß verwendbaren Wirkstoffe geht aus den nachfolgenden Beispielen hervor.

Anwendungsbeispiele

In den folgenden Anwendungsbeispielen wird die nachstehend aufgeführte Verbindung als Vergleichsubstanz herangezogen:

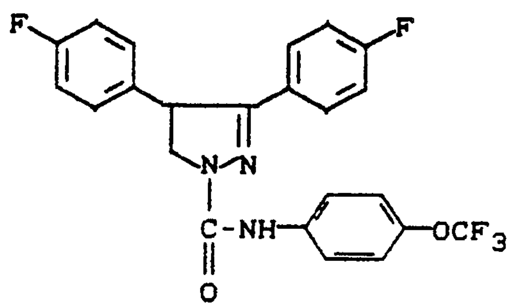

(A)

3,4-(4-Fluorphenyl)-1-(4-trifluormethoxyphenyl )-carbamoylpyrazolin (bekannt aus EP 0 267 869).

Beispiel A

Erysiphe-Test (Gerste) / kurativ

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp.hordei bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 4, 1, 2, 3.

## Tabelle A

**Erysiphe-Test (Gerste) / kurativ**

| Wirkstoff | Wirkstoffkonzentration in der Spritzbrühe in Gew.-% | Wirkungsgrad in % der unbehandelten Kontrolle |
|---|---|---|
| 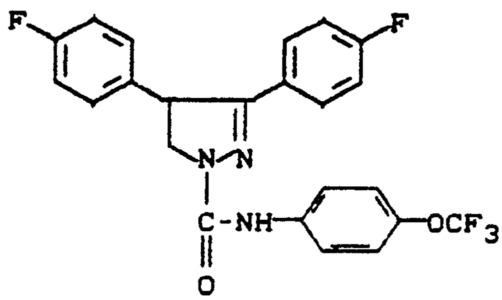 (A) bekannt | 0,025 | 50 |
| 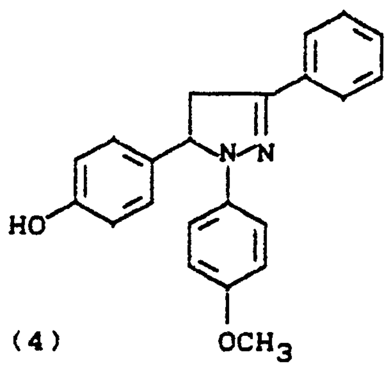 (4) | 0,025 | 96 |
| 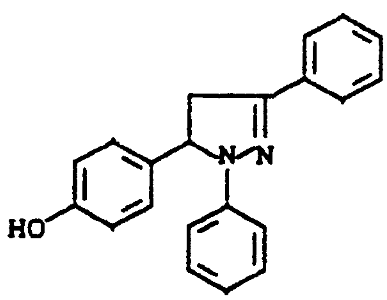 (1) | 0,025 | 88 |

**Tabelle A** (Fortsetzung)

Erysiphe-Test (Gerste) / kurativ

| Wirkstoff | Wirkstoff-konzentration in der Spritzbrühe in Gew.-% | Wirkungsgrad in % der unbehandelten Kontrolle |
|---|---|---|
| 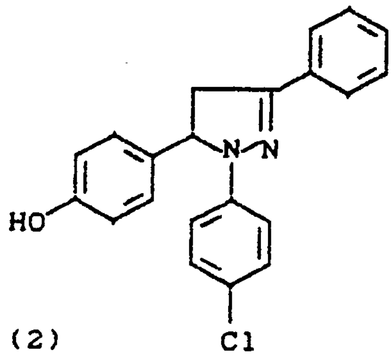 (2) | 0,025 | 100 |
| 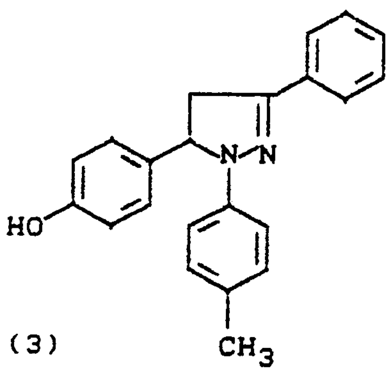 (3) | 0,025 | 84 |

Beispiel B

Erysiphe-Test (Weizen) / protektiv

Lösungsmittel: 100 Gewichtsteile Dimethylformamid Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20 °C und einer relativen Luftfeuchtigkeit van ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß der Herstellungsbeispiele: 4 und 3.

**Tabelle B**

**Erysiphe-Test (Weizen) / protektiv**

| Wirkstoff | Wirkstoffkonzentration in der Spritzbrühe in Gew.-% | Wirkungsgrad in % der unbehandelten Kontrolle |
|---|---|---|
| 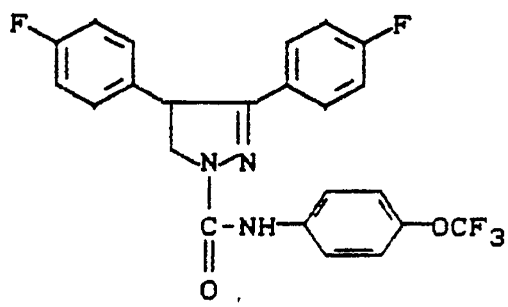 (A) bekannt | 0,025 | 0 |
| 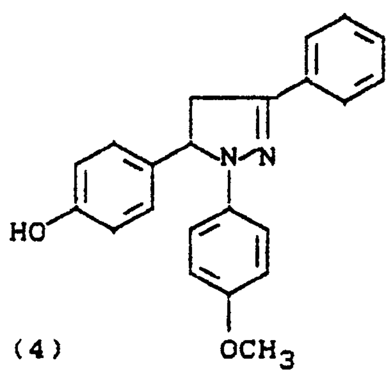 (4) | 0,025 | 100 |
| 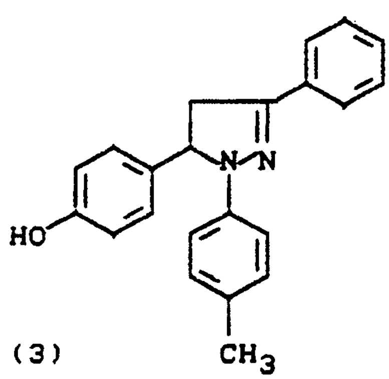 (3) | 0,025 | 100 |

Herstellungsbeispiele

Beispiel 1

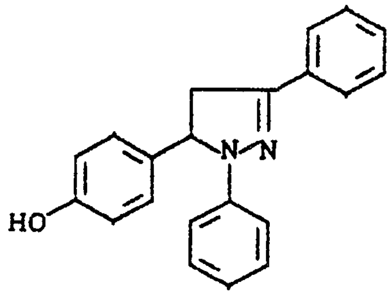

2,24 g (10,0 mMol) 1-Phenyl-3-(4-hydroxyphenyl)-2-prapen-1-on werden bei Raumtemperatur in Eisessig suspendiert und mit 1,08 g (10,0 mMol) Phenylhydrazin versetzt. Das Reaktionsgemisch wird langsam auf 100° C erhitzt und 2 Stunden bei dieser Temperatur gehalten. Beim Abkühlen kristallisiert das Reaktionsprodukt aus. Zur Reinigung löst man das Produkt in 30 ml Essigsäureethylester, wäscht mit Natriumhydrogencarbonat-Lösung und anschließend mit Wasser. Nach Trocknung über Natriumsulfat wird die Lösung eingeengt, wobei das Produkt auskristallisiert. Man erhält 1,80 g (57 % der Theorie) 1,3-Diphenyl-5-(4-hydroxy-phenyl)-2-pyrazolin vom Schmelzpunkt 144° C - 145° C.

In entsprechender Weise und gemäß den allgemeinen Herstellungsangaben erhält man die nachfolgend in Tabelle 1 aufgeführten Verbindungen.

<u>**Tabelle 1**</u>

| Beispiel Nr. | $R^1$ | $R^2$ | $R^3$ | Schmelzpunkt |
|---|---|---|---|---|
| 2 | 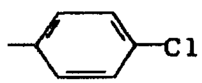 | 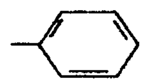 | 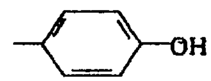 | 174-175° C |
| 3 | 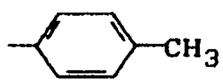 | 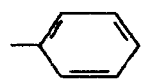 | 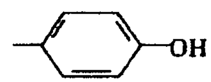 | 102-103° C |
| 4 | 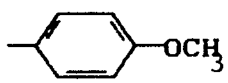 | 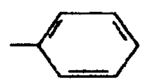 | 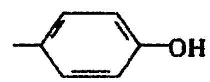 | 171-172° C |
| 5 | 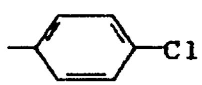 | 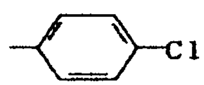 | 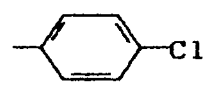 | 142-144° C |
| 6 | 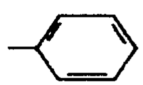 | 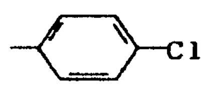 | 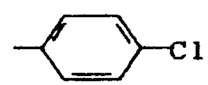 | 140-142° C |

**Tabelle 1** (Fortsetzung)

| Beispiel Nr. | $R^1$ | $R^2$ | $R^3$ | Schmelzpunkt |
|---|---|---|---|---|
| 7 | 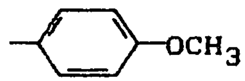 | 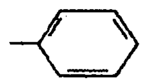 | 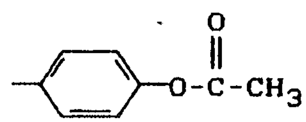 | 66-68° C |
| 8 | 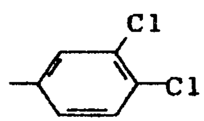 | 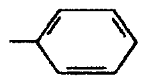 | 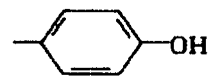 | Öl |
| 9 | 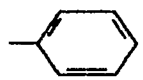 | 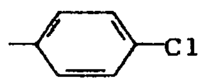 | 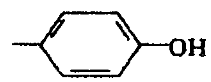 | 153-155° C |
| 10 | 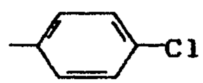 | 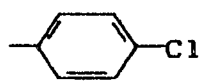 | 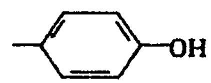 | Öl |
| 11 | 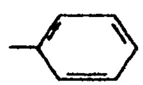 | 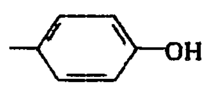 | 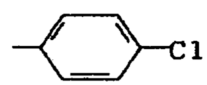 | 159-160° C |

<u>Tabelle 1</u> (Fortsetzung)

| Beispiel Nr. | $R^1$ | $R^2$ | $R^3$ | Schmelzpunkt |
|---|---|---|---|---|
| 12 | 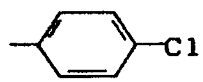 | 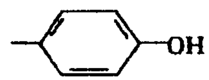 | 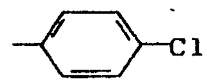 | 126-128° C |
| 13 | 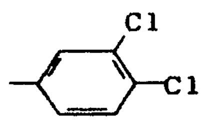 | 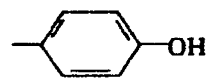 | 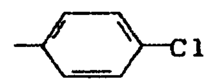 | 68-70° C |
| 14 | 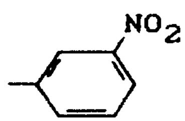 | 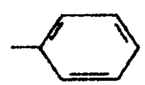 | 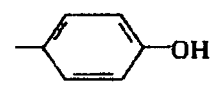 | 178-180° C |
| 15 | 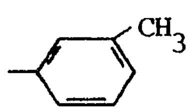 | 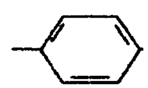 | 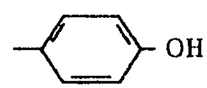 | 157-158° C |

**Tabelle 1** (Fortsetzung)

| Beispiel Nr. | $R^1$ | $R^2$ | $R^3$ | Schmelzpunkt |
|---|---|---|---|---|
| 16 | 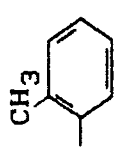 | 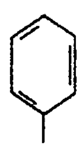 | 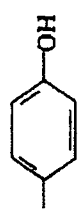 | 190-192° C |
| 17 | 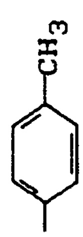 | 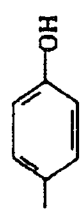 | 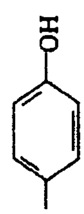 | 238-240° C |
| 18 | 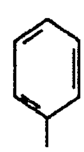 | 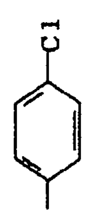 | 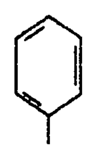 | 139-141° C |

**Ansprüche**

1. Verwendung von 1,3,5-Triaryl-2-pyrazolin-Derivaten der allgemeinen Formel (I),

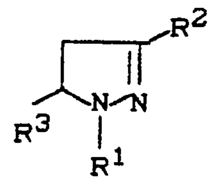

(I)

in welcher

$R^1$, $R^2$ und $R^3$ unabhängig voneinander jeweils für gegebenenfalls einfach oder mehrfach, gleich oder verschieden substituiertes Phenyl stehen, wobei als Substituenten in Frage kommen:

Halogen, Hydroxy, Mercapto, Nitro, Amino, Sulfo ($-SO_3H$), geradkettiges oder verzweigtes Alkyl mit 1 bis 4 Kohlenstoffatomen, geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen, geradkettiges oder verzweigtes Alkoxy oder Alkylthio mit jeweils 1 bis 4 Kohlenstoffatomen, (Di)alkylamino mit 1 bis 4 Kohlenstoffatomen in den jeweiligen geradkettigen oder verzweigten Alkylteilen, Carboxyl, geradkettiges oder verzweigtes Alkoxycarbonyl mit 1 bis 8 Kohlenstoffatomen im Alkoxyteil, geradkettiges oder verzweigtes Acyloxy mit 1 bis 8 Kohlenstoffatomen, weiterhin kommen als Substituenten jeweils gegebenenfalls einfach oder mehrfach, gleich oder verschieden substituiertes Phenyl, Phenoxy oder Phenoxycarbonyl in Frage, wobei als Substituenten die oben aufgeführten Substituenten für Phenyl genannt seien, deren Isomere oder Isomerengemische zur Bekämpfung von Pilzen.

2. Verwendung von 1,3,5-Triaryl-2-pyrazolin-Derivaten der Formel (I) gemäß Anspruch 1,

in welcher

$R^1$, $R^2$ und $R^3$ unabhängig voneinander jeweils für gegebenenfalls einfach bis dreifach, gleich oder verschieden substituiertes Phenyl stehen, wobei als Substituenten in Frage kommen:

Fluor, Chlor, Brom, Hydroxy, Mercapto, Nitro, Amino, Sulfo ($-SO_3H$), geradkettiges oder verzweigtes Alkyl mit 1 bis 3 Kohlenstoffatomen; Halogenalkyl, Halogenalkoxy oder Halogenalkylthio mit jeweils 1 oder 2 Kohlenstoffatomen und jeweils 1 bis 5 Fluor- und/oder Chloratomen, geradkettiges oder verzweigtes Alkoxy oder Alkylthio mit jeweils 1 bis 3 Kohlen stoffatomen, Alkylamino oder Dialkylamino mit 1 oder 2 Kohlenstoffatomen in den jeweiligen Alkylteilen, Carboxyl, geradkettiges oder verzweigtes Alkoxycarbonyl mit 1 bis 6 Kohlenstoffatomen im Alkoxyteil, geradkettiges oder verzweigtes Acyloxy mit 1 bis 6 Kohlenstoffatomen, weiterhin kommen als Substituenten jeweils gegebenenfalls einfach bis dreifach, gleich oder verschieden substituiertes Phenyl, Phenoxy oder Phenoxycarbonyl in Frage, wobei als Substituenten die als bevorzugt für Phenyl aufgeführten Substituenten genannt seien, deren Isomere oder Isomerengemische, zur Bekämpfung von Pilzen.

3. Verwendung von 1,3,5-Triaryl-2-pyrazolin-Derivaten der Formel (I) gemäß Anspruch 1, in welcher

$R^1$, $R^2$ und $R^3$ unabhängig voneinander jeweils für gegebenenfalls einfach oder zweifach, gleich oder verschieden substituiertes Phenyl stehen, wobei als Substituenten in Frage kommen:

Fluor, Chlor, Brom, Hydroxy, Mercapto, Nitro, Amino, Sulfo ($-SO_3H$), Methyl, Ethyl, Trifluormethyl, Trifluormethoxy, Trifluormethylthio, Methoxy, Methylthio, Methylamino, Dimethylamino, Carboxyl, Methoxycarbonyl, Ethoxycarbonyl, n- oder i-Propoxycarbonyl, n-, s-, i-oder t-Butoxycarbonyl, Formyloxy, Acetyloxy, Propionyloxy, n-Butyryloxy oder i-Butyryloxy, weiterhin kommen als Substituenten jeweils gegebenenfalls einfach oder zweifach, gleich oder verschieden substituiertes Phenyl, Phenoxy oder Phenoxycarbonyl in Frage, wobei als Substituenten die als besonders bevorzugt aufgeführten Substituenten für Phenyl genannt seien, deren Isomere und Isomerengemische zur Bekämpfung von Pilzen.

4. Schädlingsbekämpfungsmittel, gekennzeichnet durch einen Gehalt an mindestens einem 1,3,5-Triaryl-2-pyrazolin-Derivat der Formel (I) nach den Ansprüchen 1 bis 3.

5. Verfahren zur Bekämpfung von Pilzen, dadurch gekennzeichnet, daß man 1,3,5-Triaryl-2-pyrazolin-Derivate der Formel (I) nach den Ansprüchen 1 bis 3 auf Pilze und/oder ihren Lebensraum einwirken läßt.

6. Verfahren zur Herstellung von Schädlingsbekämpfungsmitteln, dadurch gekennzeichnet, daß man 1,3,5-Triaryl-2-pyrazolin-Derivate der Formel (I) nach den Ansprüchen 1 bis 3 mit Streckmitteln und/oder oberflächenaktiven Mitteln vermischt.

7. 1,3,5-Triaryl-2-pyrazolin-Derivate der allgemeinen Formel (IA)

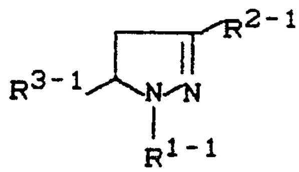

(IA)

in welcher
$R^{1-1}$ für 4-Chlorphenyl, 4-Methylphenyl oder 4-Methoxyphenyl steht,
$R^{2-1}$ für Phenyl steht und
$R^{3-1}$ für 4-Hydroxyphenyl steht, deren Isomere oder Isomerengemische.

8. Verfahren zur Herstellung von 1,3,5-Triaryl-2-pyrazolin-Derivaten der Formel (IA)

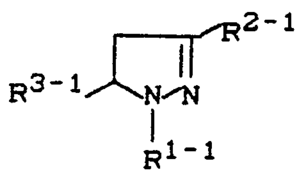

(IA)

in welcher
$R^{1-1}$ für 4-Chlorphenyl, 4-Methylphenyl oder 4-Methoxyphenyl steht,
$R^{2-1}$ für Phenyl steht und
$R^{3-1}$ für 4-Hydroxyphenyl steht,
dadurch gekennzeichnet, daß man Enone der Formel (II)

$$R^{2-1}-\overset{O}{\overset{\|}{C}}-CH=CH-R^{3-1} \quad (II)$$

mit Hydrazinen der Formel (III)
$H_2N-NH-R^{1-1}$ (III)
in welchen
$R^{1-1}$, $R^{2-1}$ und $R^{3-1}$ die oben angegebenen Bedeutungen haben, gegebenenfalls in Gegenwart eines Verdünnungsmittel und gegebenenfalls in Gegenwart eines Katalysators kondensiert.

Europäisches Patentamt

# EUROPÄISCHER RECHERCHENBERICHT

Nummer der Anmeldung

EP 90 11 3022

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A-1 158 295 (BAYER AG)<br>--- | | C 07 D 231/06<br>A 01 N 43/56 |
| D,A | DE-A-2 550 548 (BASF AG)<br>--- | | |
| D,A | CH-A- 609 977 (SANDOZ AG)<br>--- | | |
| D,A | DE-A-2 644 286 (HENKEL KGAA)<br>--- | | |
| D,A | DE-A-2 749 982 (RICOH CO., LTD)<br>----- | | |
| | | | RECHERCHIERTE SACHGEBIETE (Int. Cl.5) |
| | | | C 07 D 231/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 13-09-1990 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)